# EUROPEAN PATENT APPLICATION

(11) **EP 4 137 122 A1**
(43) Date of publication of application: **22.02.2023**
(21) Application number: 21201234.8
(22) Date of filing: 06.10.2021
(51) Int. Cl.: A61K 9/00, A61K 9/16, A61K 31/192

(54) **IMPROVED MANUFACTURING PROCESS**

(30) Priority: 19.08.2021 WO PCT/CN2021/113487
(71) Applicant: Wyeth Pharmaceutical Co., Ltd., Suzhou, Jiangsu 215128 (CN)
(72) Inventor: Khan, Majed Mohammed, 122001 Haryana (IN); Jiang, Shaobo, Suzhou, 215128 (CN)
(74) Representative: Haleon Patent Department

(57) **Abstract**

The present invention relates to improved methods and processes for producing ibuprofen arginine salt and particles or granules thereof. The invention further relates to soluble solid free-flowing compositions comprising Ibuprofen-arginine salt and sucrose, particularly pharmaceutical compositions comprising same, in the form of water-soluble powders and granules.

## Description

### Field of the Invention

The present invention relates to improved methods and processes for producing ibuprofen arginine salt and particles or granules thereof. The invention further relates to soluble solid free-flowing compositions comprising Ibuprofen-arginine salt and sucrose, particularly pharmaceutical compositions in the form of water-soluble powder or granules.

### Background of the Invention

Ibuprofen, also known as isobutylphenylpropionic acid, is an active pharmaceutical ingredient (API) belonging to the non-steroidal anti-inflammatory drug (NSAIDs) class. It is widely used in non-prescription and prescription medicines for its efficient analgesic and anti-inflammatory properties. Producing ibuprofen in soluble pharmaceutical compositions can be problematic and thus carefully optimised manufacturing processes are needed. Formulation of ibuprofen free acid into a water-soluble granule form, in particular suitable for oral dosage pharmaceutical compositions, is complicated due to its poor water solubility. The solubility of ibuprofen is also highly pH dependent resulting in reduced bioavailability and thus delayed analgesic and anti-inflammatory action. This is due to absorption occurring primarily in the intestinal tract as opposed to the stomach.

To overcome the challenge of water-solubility and thus bioavailability, ibuprofen free acid may be converted into a highly water-soluble salt form by saltification of ibuprofen with, for example, basic amino acids such as lysine or arginine or salts such as sodium or potassium. In so doing, ibuprofen salt formulations provide fast absorption and fast action as compared to conventional formulations comprising ibuprofen free acid.

KR100730393 discloses a method for preparing a combined preparation of ibuprofen and arginine, comprising the steps of: preparing a solution containing ibuprofen and arginine; and preparing granules by spraying the solution. The solution may be prepared by dissolving ibuprofen and arginine in a predetermined amount of a solvent such as methanol, ethanol, acetone, isopropyl alcohol, methylenchloride, purified water, etc.. The solution is sprayed onto a pharmaceutically acceptable excipient, such as non-crystal cellulose, corn starch and colloidal silicon dioxide.

To produce commercially viable pharmaceutical compositions comprising said ibuprofen arginine salt granules, additional processes are needed.

### Summary of the Invention

The present invention is directed towards improved processes for producing particles or granules comprising ibuprofen-arginine salt.

The processes are generally simpler than previous processes and/or more energy efficient. Advantageously, the processes may be performed at lower temperatures whilst also avoiding the use of chemical solvents or strong acids and bases. As such the processes facilitate production of water-soluble ibuprofen-arginine salt whilst avoiding the need for time-consuming purification or processing steps. The particles or granules produced are particularly suitable for use in water soluble compositions, for example water soluble pharmaceutical compositions, having characteristics making them more amenable to dissolution in water thereby providing (when consumed) a rapid onset of analgesic action.

In a First Aspect of the Invention, there is provided a process for producing a water-soluble ibuprofen-arginine salt, particularly in the form of particles or granules.

Particularly the process is a spray drying process for producing a solid free-flowing composition of granules consisting of Ibuprofen-arginine salt and sucrose, said process comprising:
a) Preparing an aqueous solution consisting of ibuprofen, L-arginine and water; and
b) spraying the aqueous solution into a fluidized bed of sucrose carrier particles under suitable conditions to form granules.

Particularly, the particles or granules are substantially dry and consist of sucrose, ibuprofen and L-arginine in free and/or salt form, more particularly the particles or granules contain less than 2% free ibuprofen.

In a Second Aspect of the Invention, there is provided a process for producing a water-soluble pharmaceutical composition, the process comprising the step of blending particles or granules produced according to the First Aspect with at least one pharmaceutical excipient.

Particularly, the at least one pharmaceutical excipient is a water-soluble pharmaceutical excipient. More particularly, the at least one excipient is selected from the group consisting of sodium bicarbonate, aspartame, sodium saccharine and flavouring. Yet more particularly, the at least one excipient consists of sodium bicarbonate, aspartame, sodium saccharine and flavouring. Still yet more particularly, the pharmaceutical composition is a water-soluble solid free-flowing water-soluble composition.

### Brief description of Figures

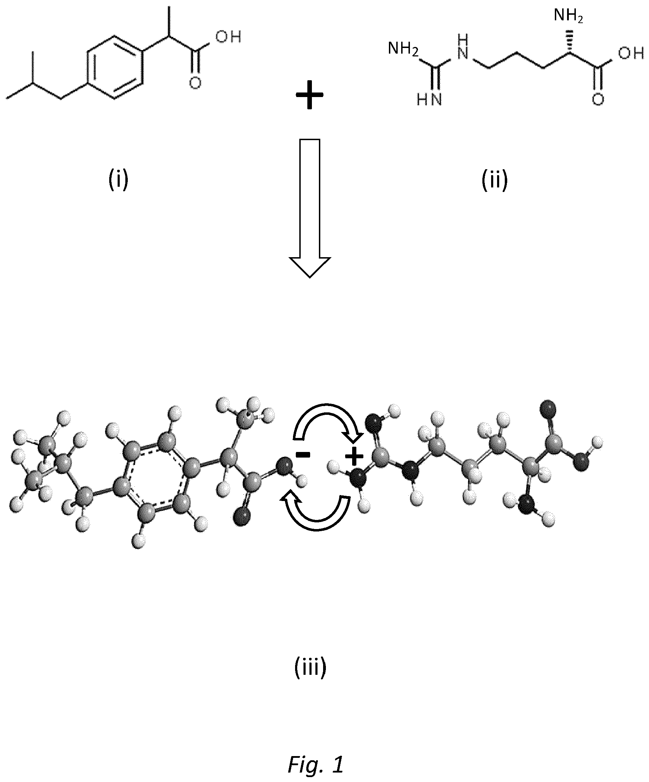

- **Figure 1.**: Shows the structure of (i) free Ibuprofen and (ii) L-arginine. When dissolved in water, ionic interactions between the carboxyl group of Ibuprofen and the guanidine group of Arginine give rise to formation of an Ibuprofen Arginine Ionic complex which upon spray drying converts to powdered salt (iii).

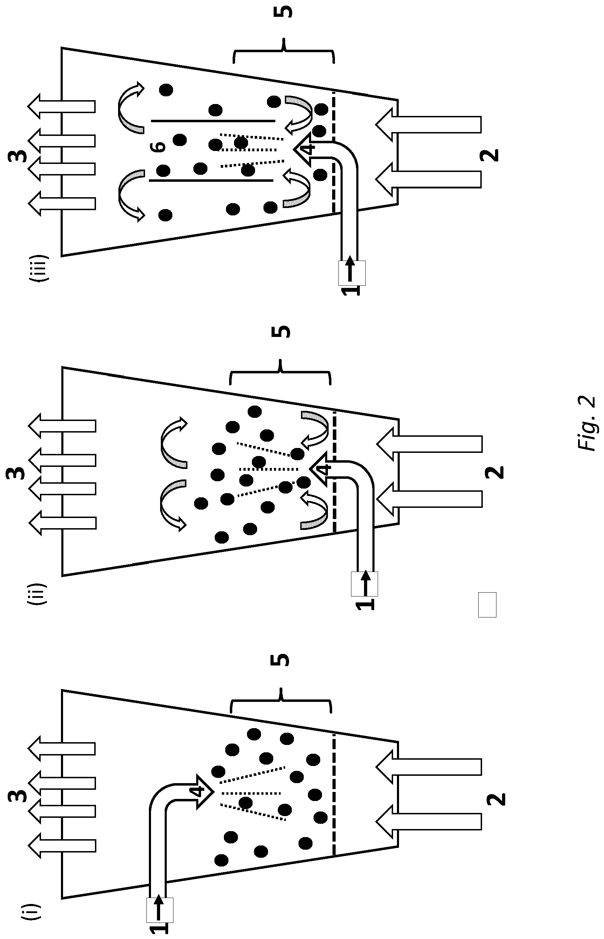

- **Figure 2.**: Provides a simplified view of (i) top-spray dryer with integrated fluid bed, (ii) bottom-spray dryer with integrated fluid bed and (iii) bottom-spray dryer with integrated fluid bed and Wurster Column insert. 1=aqueous solution fluid feed in; 2= Air Inflow; 3=Air Outflow; 4=Spray nozzle; 5=Fluid Bed; 6=Wurster Column; ●=Sucrose carrier particles.

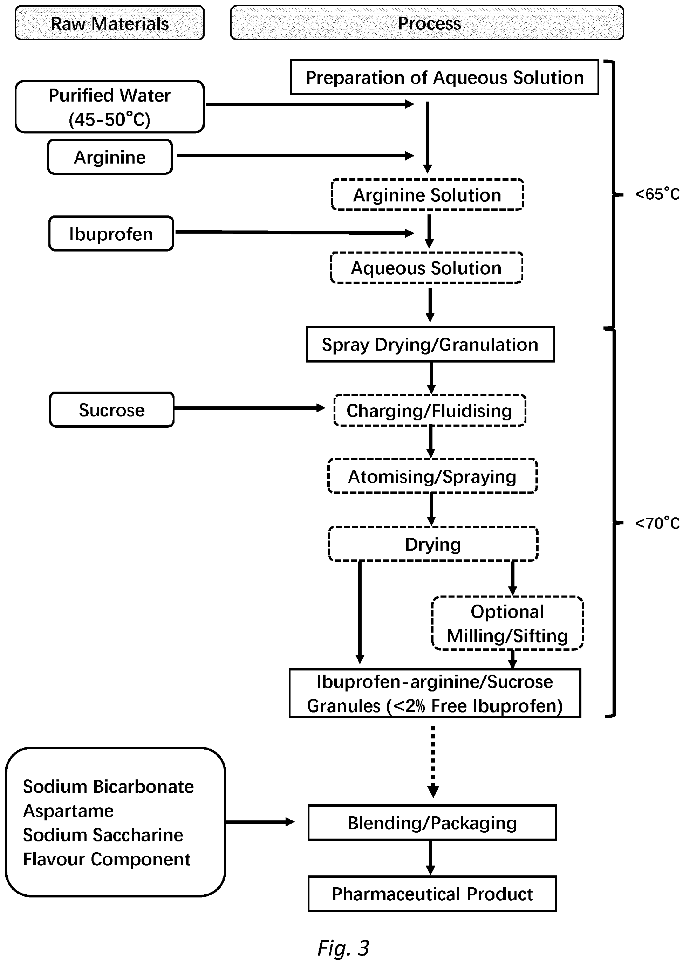

- **Figure 3.**: Provides a simplified overview of the low temperature (<70°C) simplified process of the invention with process intermediates and intermediate steps indicated by dashed lines. Optional downstream steps of blending and packaging granules prepared by the processes of the invention into a pharmaceutical product are also shown.

### Description of the Invention

Conventional processes for the preparation and granulation of ibuprofen salts are often time-consuming, multi-step processes. Often these methods utilize organic, chemical and other solvents, strong acids/bases, or high temperature processing steps. The Inventors have developed optimized processes for the preparation of ibuprofen-arginine salt in the form of particles or granules having improved solubility. The processes of the invention utilize water as the sole solvent and do not require the use of chemical, organic or other solvents that can be difficult to remove. Surprisingly, even though the processes utilize water as the sole solvent, they may be performed at lower temperatures (generally less than 70°C) without the need for costly or overly complex equipment set-ups. In addition, the processes may be performed in a continuous manner further lending to efficiency. The particles or granules produced are particularly suitable for use in water soluble compositions, for example water soluble pharmaceutical compositions, having low levels of free ibuprofen (i.e. <2%) and physical characteristics (such as an increased porosity or particle surface area) making them more amenable to dissolution in water thereby providing (when consumed) a rapid onset of analgesic action.

### Ibuprofen/l-arginine/ibuprofen-arginine salt

Ibuprofen ((R,S)-2-(4-isobutylphenyl)propionic acid) is a poorly soluble non-steroidal anti-inflammatory drug (NSAID). The solubility of Ibuprofen is highly pH dependent, increasing above pH 6.5, such that it is primarily absorbed in the intestinal tract (rather than the stomach) leading to delayed onset of analgesic action.

Since Ibuprofen is weakly acidic, solubility and bioavailability may be increased by saltification with, for example, lysine or arginine. Following oral administration, Ibuprofen salts generally exhibit an improved pharmacokinetic profile of absorption when compared to the free acid form of ibuprofen, having reduced Tₘₐₓ (the time taken to reach maximum concentration) and increased Cₘₐₓ (the maximum concentration).

The present invention relates to the pharmaceutically acceptable salt Ibuprofen-arginine, also referred to as Ibuprofen arginate (CAS No.: 57469-82-6), formed as the product of a reaction between the carboxyl group of Ibuprofen (CAS No.: 15687-27-1) and the guanidine group of the amino acid L-arginine (CAS No.: 74-79-3) (Figure 1).

The process of the Invention is a spray drying process for producing a solid free-flowing composition of granules consisting of Ibuprofen and L-arginine, substantially in salt form as Ibuprofen-arginine salt, and sucrose.

Spray drying is known in the art and the processes of the Invention may be performed with commercially available equipment. Particularly, the processes of the present invention utilize spray-drying in combination with a fluidized bed, preferably, a spray dryer with an integrated fluid-bed. Particularly the processes utilize a spray dryer with (i) means for top spray, bottom spray or tangential spray and (ii) an integrated fluid-bed apparatus. Particularly the means for top spray or bottom spray is at least one nozzle configured for top spray or bottom spray respectively. In other embodiments, a spray dryer with an integrated fluid-bed apparatus equipped with at least one nozzle configured for tangential spray may be used. A spray dryer with an integrated fluid-bed apparatus equipped with at least one nozzle configured for either top spray or bottom spray is preferred.

### Preparation of aqueous solution

In a first step an aqueous solution consisting of ibuprofen, L-arginine and water is prepared. The term "aqueous" refers to the use of water as a solvent and reference to an "aqueous solution" described herein refers to solutions that utilise water as the sole (only) solvent. Aqueous solutions are predominantly water, for example at least about 55% w/w, such as from 55% w/w to 80% w/w, from about 58% w/w to about 75% w/w or from about 58% w/w to about 72% w/w, retaining the solution characteristics of water. Particularly, aqueous solutions of the invention comprise less than 45% w/w dissolved solids, for example, 42% w/w or less, such as between about 28% w/w to about 42% w/w dissolved solids. In this context, "dissolved solids" refers to the total amount, in w/w, of Ibuprofen, L-arginine and Ibuprofen-arginine salt.

The aqueous solution is prepared by dissolving L-arginine and Ibuprofen in water. Particularly, the aqueous solution is prepared using a molar excess of L-arginine to ibuprofen. A molar excess is advantageous since it promotes formation of the ibuprofen-arginine salt ensuring that substantially all of the ibuprofen will react with L-arginine, thereby minimizing free ibuprofen in the final composition. Particularly, the ratio of ibuprofen to L-arginine in the aqueous solution is from about 1:1.05 mole to about 1:1.15 mole, for example, about 1:1.05, about 1:1.06, about 1:1.07, about 1:1.08, about 1:1.09, about 1:1.1, about 1:1.11, about 1:1.12, about 1:1.13, about 1:1.14 or about 1:1.15. In certain embodiments the molar ratio is about 1:1.1.

The required amounts of L-arginine and ibuprofen are dissolved in water, particularly pharmaceutical grade water, for example, purified through distillation, deionization, reverse osmosis or equivalent. In some embodiments, water for injection is used. "water for injection" means water suitable for injection, particularly as defined according to the European Pharmacopoeia, the United States Pharmacopoeia, or the Chinese Pharmacopoeia. In some embodiments the water has at least one, or preferably all, of the following characteristics determined using standard methods known in the art: Total organic carbon (TOC) ≤0.50, conductivity ≤1.1 (20°C), Nitrate (NO₃) ≤0.2ppm, aerobic bacteria ≤10 CFU/100ml and bacterial endotoxins ≤0.25 IU/mL.

The aqueous solution is prepared using water having a temperature of less than 70°C, less than 65°C, particularly from about 45°C to about 60°C, such as from about 45°C to about 55°C, or from about 45°C to about 50°C, for example, 45°C, 46°C, 47°C, 48°C, 49°C or 50°C.

Particularly the aqueous solution consisting of ibuprofen, L-arginine and water is prepared by first adding the L-arginine to the water and allowing it to dissolve thereby forming an aqueous solution of L-arginine, and then adding the Ibuprofen to the aqueous solution of L-arginine thereby forming the aqueous solution consisting of ibuprofen, L-arginine and water. More particularly, the L-arginine is added to the water and stirred until a clear solution is obtained (indicating that the L-arginine is fully dissolved) thereby forming an aqueous solution of L-arginine. Next, the Ibuprofen is added to the aqueous solution of L-arginine and stirred, thereby forming the aqueous solution consisting of ibuprofen, L-arginine and water.

The term 'consisting of' is used to mean that only ibuprofen, L-arginine and water are used in the preparation of the aqueous solution (and nothing else is added). However, and for the avoidance of doubt, the skilled person will understand that the Ibuprofen and L-arginine will naturally interact in the aqueous solution to form an ionic complex of the Ibuprofen-arginine salt. Thus, use of the term 'consisting of' in this context encompasses the presence of ibuprofen and L-arginine in their free or salt form, i.e. as ibuprofen-arginine salt (wherein the salt form is produced directly in the aqueous solution as a reaction product within the aqueous solution).

Once the aqueous solution consisting of ibuprofen, L-arginine and water has been prepared, it will be incubated for a time sufficient for substantially all of the ibuprofen to react with L-arginine to form ibuprofen-arginine salt. Particularly the aqueous solution will be stirred whilst it is incubated. Particularly, the aqueous solution is incubated at a temperature of less than 70°C, particularly at less than 65°C, particularly from about 45°C to about 60°C, such as from about 45°C to about 55°C, or from about 45°C to about 50°C, for example, 45°C, 46°C, 47°C, 48°C, 49°C or 50°C. More particularly, during incubation the temperature is maintained at a temperature of about 45°C to about 60°C, such as from about 45°C to about 55°C, or from about 45°C to about 50°C, for example, 45°C, 46°C, 47°C, 48°C, 49°C or 50°C. Yet more particularly, the temperature of the water used to prepare the aqueous solution is substantially the same as the temperature used for incubation. Still yet more particularly, the water and aqueous solution are maintained at a substantially constant temperature of from about 45°C to about 60°C for the duration or step (a). A skilled person will understand that even though a temperature controller is set to maintain a certain temperature, the actual temperature obtained will inevitably fluctuate to a certain extent. The term, "substantially constant temperature" is used to refer to a temperature that does not fluctuate upwardly or downwardly from the desired temperature by more than 1 °C, particularly by no more than 0.5 °C, more particularly ±0.01 °C or even more particularly ±0.005 °C.

The time sufficient for substantially all of the Ibuprofen to react with L-arginine to form ibuprofen-arginine will vary depending on scale, i.e. volume of solution. Particularly the aqueous solution is incubated and/or stirred until a clear solution is obtained (indicating that the L-arginine and Ibuprofen have fully reacted with each other to form Ibuprofen-arginate). More particularly, the aqueous solution is incubated and/or stirred for up to 1.5 hours, for example, up to about an hour, such as from 30 minutes to about 1 hour. Yet more particularly, the aqueous solution is incubated and/or stirred for at least 30 minutes to about 1 hour until a clear solution is obtained.

More particularly, the aqueous solution is incubated for a time sufficient for substantially all of the ibuprofen to react with L-arginine thereby forming ibuprofen-arginine salt such that the aqueous solution comprises less than 2% free ibuprofen, for example less than 1.9%, less than 1.8%, less than 1.7%, less than 1.6% or less than 1.5% free ibuprofen, such as about 1.6%, about 1.5%, about 1.4%, about 1.3%, about 1.2%, about 1.1% or about 1.0% free ibuprofen. The term "free Ibuprofen" refers to Ibuprofen that is not associated with L-arginine as a salt. The percentage of free Ibuprofen may be determined using standard assays known in the art, for example, using chromatographic or spectrofluorometric assays. Particularly, the percentage of free Ibuprofen is determined using High-performance liquid chromatography (HPLC).

In certain embodiments, the aqueous solution is incubated and/or stirred for 30 minutes to 1 hour until a clear solution is obtained that comprises less than 2% free ibuprofen, particularly as determined by HPLC. In certain embodiments, the aqueous solution is incubated and/or stirred at a temperature of 45°C to 50°C for 30 minutes to 1 hour until a clear solution is obtained that comprises less than 2% free ibuprofen.

Following completion of step (a) the aqueous solution is used directly for step (b) without the need for any intermediate processing steps such as filtration, purification and the like. More particularly, following completion of step (a) the aqueous solution is used directly as the feed solution for step (b) without any intervening process steps.

### Spray-drying/granulation

The second step of the method comprises (b) spraying the aqueous solution into a fluidized bed of sucrose carrier particles under suitable conditions to form granules. Particularly a spray dryer with integrated fluid-bed apparatus is used (Figure 2). The aqueous solution is used as the feed solution for the spray dryer. In certain embodiments, the aqueous solution may be prepared and incubated in a feed tank connected to the spray dryer.

Fluidisation is an operation in which solid particles are transformed into a fluid like state through suspension in air (or other gas). Air (or other gas) is passed upward through a bed of fine particles at a suitable velocity such that the frictional force between particles and air (or other gas) counterbalances the particle weight. At suitable air/gas velocities, the particles behave like a fluid. A "fluidized bed of sucrose carrier particles" refers to solid particles of sucrose that have been dynamically suspended in a flow of a gas, for example air, such that the particles have a free flowing, fluid-like behavior.

Sucrose carrier particles used in the processes of the invention have a diameter of no less than 150 µm. In some embodiments, sucrose carrier particles used in the processes of the invention may have a diameter of no less than 200 µm. Sucrose carrier particles used in the processes of the invention have a diameter of no more than 450 µm. In some embodiments, sucrose carrier particles used in the processes of the invention have a diameter of no more than 300 µm. In some embodiments, sucrose carrier particles used in the processes of the invention have a diameter of no less than 150 µm and no more than 300 µm. In some embodiments, sucrose carrier particles used in the processes of the invention have a diameter of no less than 200 µm and no more than 450 µm.

Particularly the mean diameter of the sucrose carrier particles is from 150 µm to 450 µm. In some embodiments, the mean diameter of the sucrose carrier particles is from 150 µm to 300 µm. In some embodiments, the mean diameter of the sucrose carrier particles is from 200 µm to 450 µm. For non-spherical particles the term "diameter" refers to the largest cross-sectional diameter of the sucrose particle size. Particle sizes may be determined by standard processes known in the art, for example, by sieve analysis (or gradation test).

For example, particle size may be determined by sieving using, for example, Chinese sieve standards. The sucrose particles may pass through a 40-mesh screen but are trapped by a 100-mesh screen (-40 +100). In some embodiments, the sucrose carrier particles pass through a 60-mesh screen but are trapped by a 100-mesh screen (-60 +100). In some embodiments, the sucrose carrier particles pass through a 40-mesh screen but are trapped by an 80-mesh screen (-40 +80). As indicated, the mesh size of particles may be provided using a plus (+) and/or minus (-) sign to indicate that the particles are either all larger than (+) or all smaller than (-) the specified mesh size. For example, a product identified as -40 +100 mesh would contain only particles that passed through a 40-mesh screen but are retained by a 100-mesh screen. Particularly, at least 90% or more of the particles will lie within the indicated range, for example, at least 91%, at least 92%, at least 93%, at least 94%, at least 95%, at least 96%, at least 97%, at least 98%, at least 99% or substantially all of the particles.

The sucrose particles are fluidized to form a fluidized bed of sucrose carrier particles.

Preferably, starch-free, soluble sucrose particles are used. Sugar particles which contain starch, can in principle be used, but are not preferred, because the starch can hinder the sugar particles from fastly dissolving and the end product may give hazy appearance in solution form.

In more detail, sucrose carrier particles are fluidized by an upward gas stream, particularly an upward stream of atmospheric air. The atmospheric air may be filtered. The atmospheric air is heated to a temperature sufficient to obtain a product temperature of about 40°C to about 45°C. In some embodiments, the upward stream of atmospheric air is heated to no more than about 70°C, for example, from about 50°C to about 70°C, such as from about 50°C to about 65°C or from about 65°C to about 70°C. Thus, to obtain a product temperature of about 40°C to about 45°C, the upward stream of atmospheric air is heated to no more than about 70°C. In some embodiments, to obtain a product temperature of about 40°C to about 45°C, the upward stream of atmospheric air is heated to a temperature of from 50°C to 70°C. In some embodiments, to obtain a product temperature of about 40°C to about 45°C, the upward stream of atmospheric air is heated to a temperature of from 50°C to 65°C. In some embodiments, to obtain a product temperature of about 40°C to about 45°C, the upward stream of atmospheric air is heated to a temperature of from 65°C to 70°C.

The inlet air speed (measured at the point of entry) may depend on the type of equipment used and can be determined by the skilled person.

In some embodiments, the humidity of the stream of atmospheric air may be controlled and may be measured in grams of water per kilogram of air.

Next, the aqueous solution is atomized and sprayed into the fluidized bed of sucrose carrier particles under suitable conditions to form granules. The term "atomized" means that the aqueous solution is reduced to a droplet or spray form from liquid form. A single-fluid or multi-fluid nozzle, such as a two-fluid nozzle, may be used to atomise the aqueous solution. Where a single fluid nozzle is used, increased fluid pressure is applied to atomise the aqueous solution into droplets at the nozzle outlet.

A multi-fluid nozzle, such as a two-fluid nozzle, utilises compressed air to atomise the aqueous solution and is preferred.

Atomisation pressure may depend on the type of spray nozzle used and the skilled person will be able to determine the correct pressure. In some embodiments, an atomization pressure of about 0.8 bar to about 1.5 bar may be used, for example 0.8 bar, 1.0 bar, 1.1 bar, 1.2 bar, 1.3 bar, 1.4 bar or 1.5 bar. In particular embodiments, an atomization pressure of 0.8 bar or 1.0 bar is used.

In some embodiments, the spray dryer with integrated fluid-bed apparatus is equipped with at least one nozzle configured for top spray (Figure 2(i), 4). Top spray refers to the process of spraying a liquid which enters from a fluid feed (Figure 2(i), 1) in a substantially downward direction onto and/or into a bed of fluidized particles (Figure 2(i), 5). The direction of spray is counter-current to the direction of gas flow (Figure 2(i), 2) through the fluidized bed.

In other embodiments, the spray dryer with integrated fluid-bed apparatus is equipped with means for bottom spray (Figure 2(ii) and (iii)). In bottom spray processes, the aqueous solution enters from a fluid feed (Figure 2(ii), 1) and is sprayed from below the fluidised bed (Figure 2(ii), 5). The direction of spray is concurrent to the direction of gas flow (Figure 2(ii), 2; Figure 2(iii), 2) through the fluidized bed (Figure 2(ii), 5; Figure 2(iii), 5). Spray nozzles (Figure 2(ii), 4; Figure 2(iii), 4) are integrated into an upward flow of air from the fluidized bed such that they are surrounded by the sucrose particles. In certain embodiments, bottom spray processes utilize a Wurster insert, for example a Wurster column (Figure 2(iii), 6). A Wurster column is a concentric, open-ended inner cylinder suspended above the center of the perforated floor of the fluid bed within the spray dryer. A spray nozzle centered beneath the inner cylinder sprays the aqueous solution upwardly into the inner cylinder as the fluidized sucrose particles travel upwardly through the spray in the inner cylinder.

As the sucrose particles (shown as black circles in Figure 2) are sprayed with droplets of the aqueous solution, they stick together (or agglomerate) forming granules that are subsequently dried as water is removed by the stream of air (or other gas) - thereby forming a fine-grained, free flowing powder which can be collected. Air exits the spray dryer through a filtered air outflow (Figure 2(i), 3; Figure 2(ii), 3 and Figure 2(iii), 3).

As discussed above, the air is heated to allow the evaporation of water from the aqueous solution and the granules. Using at least one spray nozzle the aqueous solution is atomized and the fluidized sucrose particles are sprayed with atomized droplets of the aqueous solution. The atomized droplets of aqueous solution coat the sucrose particles causing the particles to stick together and form granules (agglomerates) as water evaporates. Particularly, the particle size distribution (PSD) of the granules produced is from 180-2000 microns (NLT 85%) as measured by sieve (using china sieve standards).

Thus, step (b) of the process may be further defined by the following phases:
(i) Charging the spray dryer chamber with sucrose carrier particles;
(ii) Fluidising the sucrose carrier particles using a stream of air;
(iii) Atomising and spraying the aqueous solution into/onto the sucrose carrier particles.

The air flow that fluidizes the sucrose particles also serves to remove water. Thus, once spraying has been completed, the air flow may be maintained to further dry the granules and the process of the invention may further comprise the step of (c) drying the granules on the fluidised bed.

Particularly the air flow is maintained at a temperature sufficient to achieve a granule temperature of from about 45°C to about 60°C, for example 45°C, 55°C or 60°C.

The moisture content of the granules produced by the process of the Invention is preferably less than 5 percent by weight loss on drying, preferably less than 3 percent by weight loss on drying, and more preferably less than 1 percent by weight loss on drying.

Loss on drying (L.O.D.) may be measured by methods disclosed in the Chinese Pharmacopeia, for example: A specified amount of sample, for example about 1 g, is placed in a tared, shallow weighing bottle and dried in an oven at 105 °C until a constant weight has been achieved (wherein the difference in the mass of the sample between two consecutive weighings (Δm) is ≤ 0.3 mg. The difference in mass of the sample before and after drying is calculated and expressed as a percentage (m/m). In some embodiments, a Fast Moisture Analyser may be used to determine L.O.D. (for example, at 105°C in around 10 minutes). In other embodiments, L.O.D. may be determined using a vacuum drying chamber (at 80 °C) to dry the sample until a constant weight is achieved.

More particularly, step (c) comprises drying the granules on the fluidised bed at about 45°C to about 60°C, such as from 45°C to 60°C or from 45°C to 55°C, until the loss on drying is less than 1% w/w.

Particularly, and in contrast to other processes in the art, the processes of the Invention are performed at temperatures below 75°C, such as 70°C or less, such 65°C or less, 60°C or less or 55°C or less. In certain embodiments, the steps of the invention are performed at temperatures within a range of from 40°C to 70°C, from 45°C to 70°C, from 45°C to 65°C or from 45°C to 60°C.

The process may further comprise the optional step of (d) milling and sifting the granules through a sieve.

A complete overview of the low temperature (<70°C) process of the invention, described above and in the Examples below, is provided in Figure 3. Process intermediates and intermediate steps are indicated using text boxes with dashed borders. The Optional downstream steps of formulating blending and packaging the granules prepared by the processes of the invention into a pharmaceutical product are also shown.

### Pharmaceutical compositions

The Invention further provides a solid spray-dried free-flowing composition of granules consisting of Ibuprofen-arginine salt and sucrose prepared by the process of the Invention. The composition may be used as a process intermediate for further blending/formulation.

Solid free-flowing granules consisting of Ibuprofen-arginine salt and sucrose produced by the process of the Invention may be mixed with at least one solid, free-flowing pharmaceutically acceptable excipient to form a solid, free-flowing water-soluble pharmaceutical composition.

Thus, the process of the Invention may further comprise a step of: blending the granules with at least one excipient to form a pharmaceutical composition and optionally packaging the pharmaceutical composition.

Preferably the pharmaceutical composition is a solid free-flowing water-soluble composition. Preferably, the at least one excipient is selected from the group consisting of sodium bicarbonate, aspartame, sodium saccharine and a flavor compound or composition.

There is also provided a solid free-flowing water-soluble composition of spray-dried granules consisting of Ibuprofen-arginine salt and sucrose, wherein the granules comprise less than 1.5% free ibuprofen, wherein at least 85% of the granules have a size of -80 +10 mesh. Particularly, the granules dissolve completely in water to form a clear solution. More particularly, a 10g sample of granules dissolves completely in 200mL water (70°C-80°C) within 5 minutes to form a clear solution or granules equivalent to unit dose when dissolved in 100 ml of water at ambient temperature (about 20°C) form a clear solution in 5 minutes.

### GENERAL

The term "comprising" encompasses "including" e.g. a composition "comprising" X may include something additional e.g. X + Y. In some implementations, the term "comprising" refers to the inclusion of the indicated active agent, such as recited polypeptides, as well as inclusion of other active agents, and pharmaceutically acceptable carriers, excipients, emollients, stabilizers, etc., as are known in the pharmaceutical industry. In some implementations, the term "consisting essentially of" refers to a composition, whose only active ingredient is the indicated active ingredient(s), for example ibuprofen-arginine salt, however, other compounds may be included which are for stabilizing, preserving, etc. the formulation, but are not involved directly in the therapeutic effect of the indicated active ingredient. Use of the transitional phrase "consisting essentially" means that the scope of a claim is to be interpreted to encompass the specified materials or steps recited in the claim, and those that do not materially affect the basic and novel characteristic(s) of the claimed invention. See, In re Herz, 537 F.2d 549, 551-52, 190 USPQ 461, 463 (CCPA 1976) (emphasis in the original); see also MPEP § 2111.03. Thus, the term "consisting essentially of' when used in a claim of this invention is not intended to be interpreted to be equivalent to "comprising". The term "consisting of" and variations thereof means including and limited to (for example, the specific recited constituents or steps). In certain territories, the term "comprising an active ingredient consisting of" may be used in place of "consisting essentially". The term "about" in relation to a numerical value x is optional and means, for example, that the numerical value may comprise some variance around the stated number to allow for routine experimental fluctuation, measurement variance or to encompass minor deviations that may achieve substantially the same results as the stated number, such as x±10%, x±5%, x±4%, x±3%, x±2% or x±1%. The word "substantially" does not exclude "completely" e.g. a composition which is "substantially free" from Y may be completely free from Y. Where necessary, the word "substantially" may be omitted from the definition of the invention. Where methods refer to process steps, for example as (a), (b), (c), etc., these are intended to be sequential, i.e., step (c) follows step (b) which is preceded by step (a).

All references or patent applications cited within this patent specification are incorporated by reference herein.

### Aspects of the Invention

The following clauses describe additional embodiments of the invention:
**Embodiment 1.** A spray drying process for producing a solid free-flowing composition of granules consisting of Ibuprofen-arginine salt and sucrose, said process comprising:
   a) Preparing an aqueous solution consisting of ibuprofen, L-arginine and water;
   b)
      (i) Charging the spray dryer chamber with sucrose carrier particles;
      (ii) Fluidising the sucrose carrier particles using a stream of air;
      (iii) Atomising and spraying the aqueous solution into and/or onto the fluidized bed of sucrose carrier particles under suitable conditions to form granules the sucrose carrier particles;
   c) drying the granules on the fluidised bed at about 45°C to about 60°C until the loss on drying is less than 1% w/w; and
   d) optionally milling and sifting the granules through a sieve;
   wherein the process is performed using a spray dryer with integrated fluid-bed apparatus equipped with at least one nozzle configured for top spray.
**Embodiment 2.** A spray drying process for producing a solid free-flowing composition of granules consisting of Ibuprofen-arginine salt and sucrose, said process comprising:
   a) Preparing an aqueous solution consisting of ibuprofen, L-arginine and water;
   b)
      (i) Charging the spray dryer chamber with sucrose carrier particles;
      (ii) Fluidising the sucrose carrier particles using a stream of air;
      (iii) Atomising and spraying the aqueous solution into and/or onto the fluidized bed of sucrose carrier particles under suitable conditions to form granules the sucrose carrier particles;
   c) drying the granules on the fluidised bed at about 45°C to about 60°C until the loss on drying is less than 1% w/w; and
   d) optionally milling and sifting the granules through a sieve;
   wherein the process is performed using a spray dryer with integrated fluid-bed apparatus equipped with at least one nozzle configured for bottom spray.
**Embodiment 3.** The process of Embodiment 1 or 2, wherein the process is performed at a temperature of less than 70°C, less than 65°C, less than 60°C or less than 55°C.

In order that this invention may be better understood, the following examples are set forth. These examples are for purposes of illustration only and are not to be construed as limiting the scope of the invention in any manner.

### Examples

### Example 1 - Wet Granulation

In order to evaluate wet granulation as a general process, a series of prototype granulations, referred to as '4.1' (batch size 1.3kg), '4.2' (batch size 6kg), '4.3' (batch size 6kg), '4.4' (batch size 2kg) and '4.5' (batch size 2kg), were prepared by high shear granulation with purified water (Table 4).

### Manufacturing Process

Prototype 4.1 was prepared by adding ibuprofen, arginine and sodium bicarbonate into a granulation bowl. The materials were mixed in the granulation bowl and hot water was added for granulation. Sucrose was added to the granulation bowl whilst the impeller and chopper were turned on.

The mixture formed a sticky mass in the granulator that was not suitable for further development.

For the preparation of further prototypes, the wet granulation process was altered: (1) Ibuprofen, arginine and sucrose were added to a granulator bowl. (2) The materials were mixed in the bowl and water was sprayed into the bowl for granulation. (3) For the final granulation, the mixture was kneaded with the impeller and chopper on. (4) The granules were dried in a fluid bed until the target LOD% was achieved.

### Results and Discussion

The process produced fine granules with a low yield within the size range between 10-80 mesh. In addition, levels of free Ibuprofen were high and the granules could not be completely dissolved in water (Table 4).

| **TABLE 4** | | | | | |
|---|---|---|---|---|---|
| **High shear granulation with purified water.** | | | | | |
| **Prototype formulations for Ibu/Arg granules.** | | | | | |
| **Ref.:** | 4.1 | 4.2 | 4.3 | 4.4 | 4.5 |
| **Batch size (kg)** | 1.3kg | 6.0 | 6.0 | 2.0 | 2.0 |
| **Water (g)** | 53 | 400 | 350 | 40 + 10 | 60 + 10 |

| | ***Results*** | | | | |
|---|---|---|---|---|---|
| | | 4.2 | 4.3 | 4.4 | 4.5 |
| **Yield (10-80 mesh)** | *Not measured due to formation of sticky mass* | 57% | 63% | NA | NA |
| **Solubility in warm water** | | ∼3 min | ∼2 min | ∼3 min | insoluble |
| **Assay of IBU** | | 96% | 90% | 95% | 140% |
| **Free IBU** | | 47% | 44% | 48% | 71% |

In conclusion, wet granulation was determined to be unsuitable for the production of ibuprofen-arginine salt granules since it was not possible to obtain uniform granules between 10-80mesh with high solubility and a low level of free ibuprofen.

### Example 2 - Top Spray Granulation

In order to evaluate top spray granulation processes, a series of prototype granules, referred to as '1.1' and '1.2' (batch size 2kg) were prepared by top spray granulation with ibuprofen and L-arginine solution (Table 1A and 1B).

### Manufacturing Process

An aqueous solution consisting of ibuprofen, L-arginine and water with a molar ratio of 1:1:1 and concentration of 42% w/wwas prepared by stirring the solution for 30 mins to 1 hour at 45°C to 50°C until a clear solution was obtained.

The spray dryer chamber was charged with sucrose carrier particles. The sucrose carrier particles were fluidized by an upward stream of atmospheric air (heated to 65°C - 70°C) having an inlet air volume of 50m³/h to 70m³/h. Following this, the aqueous solution was atomized (atomization pressure range of 1.5-2.0 bar) with a single-fluid nozzle and sprayed from the top of the chamber (spray rate range of 5.5-11 g/min) onto the fluidised bed of sucrose carrier particles.

The resulting granules were dried on the fluidised bed with temperature control to obtain a granule temperature of about 50°C to 60°C. The processing time for each batch was around 2 hours. Table 1A contains the process parameters for each batch.

| **TABLE 1A** | | |
|---|---|---|
| **Top spray granulation with ibuprofen and arginine solution.** | | |
| **Prototype formulations for IBU/ARG granules.** | | |
| ***Parameter*** | | |
| **Ref.:** | **1.1 (210305)** | **1.2 (210308)** |
| **Batch size (kg)** | 2.0 | 2.0 |
| **Atomization pressure (bar)** | 2.0 | 1.5 |
| **Spray rate (g/min)** | 5.5-11 | 5.5-11 |
| **Processing time (hrs)** | 2 | 2 |

### Results and Discussion

The percent yield was calculated by determining acceptable granule weight (amount passed through a 10-mesh sieve)/total weight of ingredients x 100.

To ensure drug efficacy, percent ibuprofen is determined by standard HPLC methods. A value within the range of 95%-105% is deemed to be acceptable.

The percentage of free ibuprofen is also determined by standard HPLC methods to ensure complete conversion of free ibuprofen to ibuprofen-arginine salt. A level of not more than 2.0% (NMT 2.0%) is used for quality control.

As shown in Table 1B, Prototype 1.1 showed a high yield of 91% with atomization pressure of 2.0 bar and spray rate 5.5-11 g/min. Using an atomization pressure of 1.5 bar for the preparation of Prototype 1.2, resulted in a lower yield of granules (85%) and ibuprofen (73%). However, levels of free ibuprofen were decreased to 1.1%, below the 2% target.

| **TABLE 1B** | | |
|---|---|---|
| Top spray granulation with ibuprofen and arginine solution. | | |
| ***Results*** | | |
| **Ref.:** | **1.1** | **1.2** |
| **Yield (10-mesh)*** | 91% | 85% |
| **Assay of IBU** | 76.8% | 73% |
| **Free IBU** | 1.3% | 1.1% |

| | | |
|---|---|---|
| *Yield% calculated by acceptable granule weight (passed through the 10-mesh)/total weight of ingredients x 100. | | |

### Example 3 - Evaluation of Sucrose Grade

To evaluate the sucrose grade, top-spray granulation was performed as before using sucrose particles sieved to obtain particular size ranges (Table 1C).

| **Table 1C** | | | | | |
|---|---|---|---|---|---|
| ***Sucrose Screening Results*** | | | | | |
| **Batch No.** | **Sucrose Grade** | **Ibu: Arg ratio** | **Yield%** | **Assay** | **Free Ibu** |
| **RD-FB01-210616** | 60-80 mesh Sucrose | 1:1.1 | 96% | 92.9% | 0.90% |
| **RD-FB01-210617** | 80-100 mesh Sucrose | 1:1.1 | 95% | 94.9% | 0.90% |
| **RD-FB01-210620** | 80-100 mesh Sucrose | 1:1.1 | 95% | 91.8% | 0.93% |

Based on the assays of ibuprofen% and percent free ibuprofen, the processes were further optimised using 80-100 mesh sucrose and a range of ibuprofen to arginine ratios, (Table 1D).

| **Table 1D** | | | | | |
|---|---|---|---|---|---|
| ***Ibu:Arg Ratio Screening Results*** | | | | | |
| **Batch No.** | **Sucrose Grade** | **Ibu: Arg ratio** | **Yield%** | **Assay** | **Free Ibu** |
| ***RD-FB01-210617*** | *80-100 mesh Sucrose* | *1:1.10* | 95% | 94.9% | *0.9%* |
| **RD-FB01-210618** | 80-100 mesh Sucrose | 1:1.05 | 90% | 94.04% | 1.0% |
| **RD-FB01-210619** | 80-100 mesh Sucrose | 1:1.15 | 96% | 95.2% | 0.8% |

### Example 4 - Bottom Spray Granulation

To evaluate an alternative to top spray granulation, a series of prototype granulations, referred to as '2.1' (batch size 1.5kg), '2.2' (batch size 1.5kg), '2.3' (batch size 1.5kg), '2.4' (batch size 1.5kg), '2.5' (batch size 1.5kg), '2.6' (batch size 2kg) and '2.7' (batch size 2kg), were prepared by bottom spray granulation using ibuprofen and L-arginine solution (Table 2A and 2B).

### Manufacturing Process

An aqueous solution consisting of ibuprofen, L-arginine and water was prepared by stirring the solution for 30 mins to 1 hour at 45°C to 50°C until a clear solution was obtained. The solution had a molar ratio of 1:1:1 and a concentration of 27.8% w/w for Prototypes 2.1 to 2.5 and 42% w/w for Prototypes 2.6 and 2.7.

The spray dryer chamber was charged with sucrose carrier particles having the characteristics listed in Table 2A. The sucrose carrier particles were fluidized with an upward stream of atmospheric air heated to 50°C to 70°C (inlet air speed of 40m³/h to 65m³/h). Next, the aqueous solution was atomized (atomization pressure range of 1.5-2.0 bar) and sprayed from the bottom of the fluidized-bed apparatus (spray rate range of 4-11 g/min) onto the fluidised bed of sucrose carrier particles with a single-fluid nozzle.

The resulting granules were dried on the fluidised bed at about 45°C to about 55°C. Processing time for the batches varied from 2-5 hours (Table 2A).

| **TABLE 2A** | | | | | | | |
|---|---|---|---|---|---|---|---|
| **Bottom spray granulation with ibuprofen and arginine solution.** | | | | | | | |
| **Prototype formulations for IBU/ARG granules.** | | | | | | | |
| ***Parameters*** | | | | | | | |
| **Ref.:** | **2.1** | **2.2** | **2.3** | **2.4** | **2.5** | **2.6** | **2.7** |
| **Batch size (kg)** | 1.5 | 1.5 | 1.5 | 1.5 | 1.5 | 2.0 | 2.0 |
| **Core** | Sugar sphere (45-60 mesh) | Sucrose | Sucrose (>60 mesh) | Sucrose (40-60 mesh) | Sucrose | Sucrose (20-60 mesh) | Sucrose (20-60 mesh) |
| **Atomization pressure (bar)** | 1.5 | 1.5 | 1.5 | 1.5 | 1.5 | 2.0 | 1.5 |
| **Spray rate (g/min)** | 6-7 | 6-7 | 6-8 | 4-8.5 | 5-7 | 5.5-11 | 5.5-11 |
| **Processing time (hrs)** | 3.5 | 3.5 | 5 | 3.5 | 5 | 2 | 2 |

### Results and Discussion

As with the top spray granulation process, the yield%, assay of ibuprofen% and free ibuprofen% were measured to assess the efficacy of the bottom spray granulation process and parameters (Table 2B).

Prototype 2.1 had high yield and ibuprofen content however, it was not completely soluble due to the presence of starch in the sugar spheres which were used.

Prototypes 2.2, 2.3 and 2.4 were optimized for the particle size distribution of sucrose and to improve the percent ibuprofen content. Free ibuprofen was relatively low in these prototypes.

In prototype 2.5, it was determined that the content of ibuprofen could be improved by spraying an coverage of the drug solution, i.e. by spraying about 10% more of the drug solution onto the fluidised bed of sucrose carrier particles (compared to the amounts used for prototypes 2.1 to 2.59. This overage compensates for a loss during the spraying process. The percentage of free ibuprofen was also low (0.3%) below the maximum 2% quality threshold.

Prototypes 2.6 and 2.7 were prepared using a higher spray rate range and a shorter processing time, also resulting in high yield and low levels of free ibuprofen (0.8% and 0.7% respectively).

| **TABLE 2B** | | | | | | | |
|---|---|---|---|---|---|---|---|
| Bottom spray granulation with ibuprofen and arginine solution. | | | | | | | |
| ***Results*** | | | | | | | |
| **Ref.:** | **2.1** | **2.2** | **2.3** | **2.4** | **2.5** | **2.6** | **2.7** |
| **Yield (10-80 mesh)** | 95% | 94% | 91% | 75% | 97% | 94% | 96% |
| **Assay of IBU** | 95% | 87% | 84% | 96% | 97% | 88% | 92% |
| **Free IBU** | 1.6% | 1.5% | 0.55% | 0.36% | 0.3% | 0.8% | 0.7% |

### Example 5 - Optimised Bottom Spray Granulation

Bottom spray granulation was performed using the optimized parameters shown in Table 3A. Briefly, the manufacturing process consisted of 1) preparation of an aqueous solution followed by 2) bottom-spray granulation.

For the aqueous spray solution of ibuprofen-arginine, 370g of L-arginine was stirred into 2000ml of warm, purified water at about 40°C until the solution was clear. 400g of ibuprofen was then added to the arginine solution and stirred until the solution was again clear.

For the granulation process, the fluid bed was pre-heated for approximately 10 minutes. Heating was stopped and 1955g of sucrose was transferred into the granulation vessel of the fluidizer. The sucrose particles were sprayed with the ibuprofen-arginine solution, starting with a low spray rate. The parameters of the apparatus were adjusted during this granulation process according to the ranges illustrated in Table 3A to maintain the appropriate fluidization state of the ingredients. The resulting granules were then dried until the LOD% was lower than 1% before being passed through a 10# and 80# mesh sieve. The granules with particle sizes between 10# and 80# mesh (acceptable granules) were weighed.

| TABLE 3A | | |
|---|---|---|
| Bottom spray granulation prototype example | | |
| Batch No. 20210112 | | |
| **Ingredients** | **Formula (mg)** | **Weight of ingredients (g)** |
| **Ibuprofen** | 400.00 | 200.00 |
| **L-arginine** | 370.00 | 185.00 |
| **Sucrose** | 1955.00 | 977.50 |
| **Purified water (conc of spraying solution about 28% (w/w)** | 2000.00 | 1000.00 |
| **Total weight** | 2725.00 | 1362.50 |

| **Parameters** | | |
|---|---|---|
| **Atomization pressure (bar)** | 1.5 | |
| **Spray rate (g/min)** | 6-8.5 | |
| **Product temperature (°C)** | 38.0-49.2- | |
| **Rotating speed (rpm)** | 8-12 | |

### Results

As illustrated in Table 3B, the yield of the target granules was 94.1%. The optimized process also produced granules having a high percentage of ibuprofen (97%) whilst the percentage of free ibuprofen was low (0.7%).

| Table 3B | | | | |
|---|---|---|---|---|
| Bottom spray granulation prototype results | | | | |
| Batch No. | lbu:Arg ratio | Yield * | Assay | Free lbu |
| 20210112 | 1:1.1 | 94.1% | 97% | 0.7% |

| | | | | |
|---|---|---|---|---|
| * Calculated as: (weight of #10-#80 mesh granules)/total weight of ingredients x 100 | | | | |

In conclusion, simplified processes based on the use of top or bottom spray-drying have been developed for the preparation of solid free-flowing granules consisting of Ibuprofen-arginine salt and sucrose. The processes produce high yield of granules having a high content of ibuprofen-arginine salt (as determined by %ibuprofen assay) whilst also having low levels of free ibuprofen.

In view of this guidance, one of ordinary skill in the art will be able to adjust apparatus and process parameters to achieve similar results with other fluid-bed/sprayer combinations that are available in the art.

## Claims

1. A spray drying process for producing a solid free-flowing composition of granules consisting of Ibuprofen-arginine salt and sucrose, said process comprising:
a) Preparing an aqueous solution consisting of ibuprofen, L-arginine and water; and
b) spraying the aqueous solution into a fluidized bed of sucrose carrier particles under suitable conditions to form granules of the sucrose carrier particles.

2. The process of claim 1, wherein the aqueous solution comprises a molar excess of L-arginine to ibuprofen.

3. The process of claim 2, wherein the ratio of L-arginine to ibuprofen in the aqueous solution is from about 1.5:1 mole to 1.05:1 mole, particularly 1.15:1 mole to 1.05:1 mole.

4. The process of claim 3, wherein the aqueous solution comprises about 1.1 mole of L-arginine per 1.0 mole of ibuprofen.

5. The process of any one of claims 1-4, wherein the water has a temperature of from 45-60°C.

6. The process of claim 5 wherein step (a) comprises (i) preparing an aqueous solution consisting of ibuprofen, L-arginine and water and (ii) incubating the solution for a time sufficient for substantially all of the ibuprofen to react with L-arginine to form ibuprofen-arginine salt.

7. The process of claim 6, wherein in step (i) the aqueous solution consisting of ibuprofen, L-arginine and water is prepared by first dissolving L-arginine in water then adding ibuprofen.

8. The process of claim 7, wherein in step (ii) the aqueous solution is incubated for up to 60 minutes.

9. The process of claim 8, wherein the suitable conditions comprise:
i. atomizing the solution into droplets using an atomization nozzle at a pressure of 0.8-1.5 bar; and/or
ii. maintaining a spray temperature of 50-65°C; and/or
iii. the sucrose carrier particles have an average particle size within the range 150µm to 450µm (#100-40 mesh), such as 150µm to 300µm or 200µm to 450µm.

10. The process of any preceding claim wherein the spraying occurs from one or more atomisation nozzles using (i) a top spray configuration or (ii) a bottom spray configuration.

11. The process of claim 9 wherein the spraying occurs from one or more atomisation nozzles using a top spray configuration.

12. The process of claim 9 wherein the spraying occurs from one or more atomisation nozzles using a bottom spray configuration.

13. The process of claim 9, 10 or 11 further comprising the step of (c) drying the granules on the fluidised bed.

14. The process of claim 12, wherein step (c) comprises drying the granules on the fluidised bed at a product temperature of 40-60°C until the loss on drying is less than 1.0% w/w.

15. The process of claim 12 or 13, further comprising the step of (d) milling and sifting the granules.

16. The process of claim 14, further comprising the step of (e) blending the granules with at least one excipient to form a pharmaceutical composition, wherein the at least one excipient is selected from the group consisting of sodium bicarbonate, aspartame, sodium saccharine and flavouring and wherein the pharmaceutical composition is a solid free-flowing water-soluble composition.

17. A solid spray-dried free-flowing composition of granules consisting of Ibuprofen-arginine salt and sucrose prepared by the process of claim 1 to 14.

18. A water soluble pharmaceutical composition prepared by the process of claim 15 or comprising the granules of claim 16.

19. A solid free-flowing water-soluble composition of spray-dried granules consisting of Ibuprofen-arginine salt and sucrose wherein the granules comprise less than 2.0% free ibuprofen, wherein at least 85% of the granules have a size of -80 +10 mesh and forms a clear solution wherein when dissolved in water.
